# EUROPEAN PATENT APPLICATION

(11) **EP 4 389 320 A1**
(43) Date of publication of application: **26.06.2024**
(21) Application number: 22214676.3
(22) Date of filing: 19.12.2022
(51) Int. Cl.: B22F 3/11, B22F 10/18, B33Y 10/00, B33Y 80/00, A61F 2/30

(54) **A METHOD FOR PRODUCING A THREE-DIMENSIONAL MICROPOROUS FILAMENT CONSTRUCT**

(71) Applicant: VITO NV, 2400 Mol (BE)
(72) Inventor: ROMBOUTS, Marleen, B-2400 Mol (BE); MULLENS, Steven, B-2400 Mol (BE)
(74) Representative: AWA Benelux

(57) **Abstract**

The present invention relates to a method for producing a three-dimensional macro-porous filament construct having interconnected microporous filaments and a suitable morphology, said method comprising the steps of:
a) preparing a mixture comprising particles of one or more metals, one or more metal alloys or a mixture of the afore mentioned materials, one or more binders, a first liquid solvent for the one or more binders, and optionally one or more dispersants;
b) dispersing particles containing at least one removable additive in said mixture;
c) depositing said mixture in the form of filaments in a predetermined three-dimensional pattern of interconnected filaments, thereby creating a three-dimensional filament-based porous green structure,
d) contacting the three-dimensional filament-based porous green structure formed in step c) with a second solvent which is a non-solvent for the one or more binders, with the purpose of inducing phase inversion thereby creating a filament-based phase inverted porous structure having a suitable filament morphology, whereby at least part of said filaments are transformed to a solid state,
e) contacting the phase inverted structure with a removal agent for removing at least part of the at least one removable additive,
f) subjecting the thus obtained structure to a thermal treatment.

## Description

### Field of the Invention

The present invention relates to a method for producing a three-dimensional macro-porous filament construct having interconnected micro-porous filaments and a suitable morphology, according to the preamble of the first claim.

The present invention also relates to a three-dimensional macro-porous filament construct thus obtained and to its use in (bio)medical products, implants, bone grafts, drug delivery devices, as well as its use in a three-dimensional catalyst, a sorbent, a heat exchange structure.

### Background of the invention

Highly porous constructs are of great importance in applications such as orthopaedic implants and bone tissue engineering. A variety of manufacturing routes have been developed for preparing polymeric, ceramic or metallic highly porous scaffolds with pore sizes ranging from 100 - 1000 µm.

An earlier patent publication by the same applicant, EP2195131, discloses a method for producing a three-dimensional micro-porous construct of filaments having two levels of porosity:
- a first level of porosity comprising interconnected macro-pores between the filaments. These macro-pores usually have an adjustable pore size distribution.
- a second level of porosity includes interconnected micro-pores within the filaments.

The method disclosed in EP2195131 involves deposition of a viscous paste in the form of filaments in a three-dimensional structure. The method in particular comprises the following steps:
a) preparing a suspension comprising particles of a predetermined metallic material such as Ti or a Ti-alloy, a liquid solvent, one or more binders and optionally one or more dispersants,
b) depositing said suspension in the form of filaments in a predetermined three-dimensional pattern thereby creating a three-dimensional filament-based porous structure,
c) inducing phase inversion whereby said filaments are transformed from a liquid to a solid state by the steps of
   c1) bringing said filaments during the deposition of the filaments into contact with a non-solvent vapour, and
   c2) immersing the structure of step c1) in a liquid non-solvent, thereby creating a filament-based porous structure having suitable filament morphology,
d) thermally treating the structure of step c) by calcining and sintering said structure.

The levels of micro-porosity achievable with this method are however limited. Although the polymeric binder interferes in the micro-pore formation and although incorporation of larger amounts of binder may be desired to increase porosity, it has been observed that pyrolysis of the polymer binder during the thermal treatment gives rise to the formation of carbon based residues. Part of these carbon residues as well as reaction products formed by the reaction of carbon with carbon-sensitive metallic material formed during the thermal treatment, may accumulate between the particles of the material forming the construct, and can interstitially be incorporated into the metal structure during thermal treatment. Especially in case the construct is made of a metallic material such as Ti or a Ti-alloy, the carbon-based residues may impart undesired brittleness to the three-dimensional construct.

There is thus a need for a method which permits manufacturing a three-dimensional porous construct with interconnected pores and with a desired, preferably an increased micro-porosity when compared to the prior art, without increasing residual impurity pickup. There is also a need for a method which permits manufacturing a three-dimensional construct comprising interconnected micro-porous filaments, which are arranged according to a desired pattern, which have a desired morphology and pore structure, and wherein the pores within the filaments are accessible from outside the construct.

The present invention therefore seeks to provide a method for manufacturing a three-dimensional macro-porous filament construct comprising interconnected micro-porous filaments with a desired morphology, wherein the pore volume represented by the micropores may be increased with respect to the prior art.

This is achieved according to the present invention with a method showing the technical features of the first claim.

Thereto, the present invention relates to a method for producing a three-dimensional macro-porous filament construct having interconnected micro-porous filaments and a suitable morphology, wherein the method comprises the steps of:
a) preparing a mixture comprising particles of one or more metals, one or more metal alloys or a mixture of the afore mentioned materials, one or more binders, a first liquid solvent for the one or more binders, and optionally one or more dispersants,
b) dispersing particles containing at least one removable additive compound in said mixture;
c) depositing said mixture in the form of filaments in a predetermined three-dimensional pattern of interconnected filaments, thereby creating a three-dimensional filament-based porous green structure,
d) contacting the three-dimensional filament-based porous green structure formed in step c) with a second solvent which is a non-solvent for the one or more binders, with the purpose of inducing phase inversion thereby creating a filament-based porous phase inverted structure having a suitable filament morphology, whereby at least part of said filaments are transformed to a solid state,
d) contacting the phase inverted structure with a removal agent for removing at least part of the at least one removable additive additive,
e) subjecting the thus obtained structure to a thermal treatment.

The inventors have surprisingly found that the incorporation of at least one removable additive into the mixture comprising particles of one or more metals, one or more metal alloys or a mixture thereof, and subsequently removing at least part of the said removable additive, permits to increase the porosity of the 3D filament construct, to an unexpected level. By incorporation and subsequent removal of the removable additive, an increase of the porosity may be achieved which exceeds the volume occupied by the at least one removable additive. In particular, micro-pores may be formed within the filaments and the porosity induced by those micro-pores may be increased to an unexpected level. An unexpected volume of micro-pores may thus be formed in the filaments.

The inventors have further found that part of the micro-pore volume may be created in step d) where solvent for the one or more binders is replaced by a non-solvent for the one or more binders and the one or more binders are caused to solidify. Part of the micro-pore volume may be formed in step e) where at least part of the removable additive is removed. Part of the micro-pore volume may be formed in step f) where a thermal treatment is carried out to achieve pyrolysis of the one or more binders. The micro-pores thereby created in step d), e) and f) are intrinsically interconnected.

Depending on the amount of removable additive incorporated in the mixture and the extent to which the removable additive is removed, a three-dimensional filament construct may be built with a porosity that extends beyond the porosity that may be obtained with phase inversion of the phase inversion binder alone. Alternatively, by incorporation of the at least one removable additive, a three-dimensional filament construct can be obtained with a same level of porosity while using lower levels of the one or more binders. Keeping the amount of the one or more binders as low as possible may be important, because the thermal treatment of step e) may cause formation of carbonaceous residues due to pyrolysis of the binder in the course of the thermal treatment, for example involving de-binding/calcination and/or sintering. At least part of this carbonaceous material may be left behind in the micro-pores of the three-dimensional construct and lead to a reduction of the mechanical properties, in particular to a reduction of the ductility and/or to an embrittlement of the construct with a risk to increasing brittleness to undesired levels. The inventors have namely observed that the removable additive is capable of interfering in the pore formation of the construct, by creating micro-porosity within the filaments at lower amounts of the one or more binders. The inventors have also observed that the removable additive is capable of increasing the porosity of the three-dimensional construct to a level that could not be obtained by the presence of the one or more binders alone.

Depending on the nature of the additive, on the desired porosity and on the intended application, removing of the removable additive in step d) to an extent as completely as possible may be carried out, or the presence of remainders of the additive may be permitted to a desired level.

Incorporation of at least one removable additive into the mixture according to this invention presents the advantage that the amount of phase inversion binder that needs to be incorporated in the mixture may be kept within limits, or alternatively the amount of phase inversion binder incorporated in the mixture may be reduced when compared to prior art mixtures, while still a desired porosity may be achieved. The present invention thus makes it possible to reduce the amount of the one or more phase inversion binders in the mixture and to still build a porous three-dimensional construct with a similar or even a higher porosity, at minimal risk to an undesired increase of the brittleness of the three-dimensional filament construct obtained after the thermal treatment. In other words, if a certain porosity is envisaged, the amount of the one or more phase inversion binders that needs to be present in the mixture to achieve a desired porosity may be reduced in comparison to the prior art, where the mixture does not contain a removable additive. Alternatively, incorporation of a removable additive according to this invention presents the advantage that a three-dimensional construct may be obtained with a porosity that extends beyond a level that could be obtained by the presence of a phase inversion binder only, i.e. in the absence of removable additive. In other words, for a same amount of phase inversion binder the porosity of the three-dimensional construct may be increased to a higher level than could be expected from the amount of removable additive present in the mixture. This is an advantage since the use of the removable additive permits to minimise formation of carbonaceous residues upon calcination and sintering.

The method of this invention presents the additional advantage that some surface roughness may be formed on an outer face of the filaments as well as on the surface of the inner pores of the filaments. Most probably this may be caused by the phase inversion of the one or more binders. Since the filaments are formed from a mixture containing the one or more binders, at least part of the one or more binders will be located on an external surface of the filaments, and phase inversion thereof may give rise to the formation of some roughness on the external surface of the filaments. A similar phenomenon will take place in the interior of the filaments, where micropores with a certain surface roughness are formed when the one or more binders are subjected to phase inversion. The presence of a certain degree of surface roughness may present significant advantages in particular when the three-dimensional construct is used as an implant or part of an implant. Surface roughness may then facilitate incorporation in the body of a living being.

Within the scope of this invention, the thermal treatment may serve the purpose of drying the construct. The thermal treatment may however also involve the removal of binder material and sintering of the construct. Normal sinter conditions can be used resulting in structures with a controllable degree of micro-porosity and pore size distribution. It is remarked that sintering may involve shrinkage of the construct, and that such shrinking may give rise to a reduction of the total porosity and average pore diameter.

The step of dispersing the at least one removable additive in the mixture of step a) comprising particles of one or more metals, one or more metal alloys or a mixture of the afore mentioned materials, one or more binders, a first liquid solvent for the one or more binders, and optionally one or more dispersants, may involve a separate step or it may be done simultaneously with preparing the mixture.

Contacting of the filaments formed in step c) with a non-solvent for the one or more binders may be done in several ways, depending on the nature of the one or more binders, the nature of the at least one removable additive and the desired micro-porosity.

According to a first preferred embodiment, the contacting of the three-dimensional filament-based porous structure with the second solvent to cause phase inversion of the one or more binders, may be carried out during deposition of the filaments. Contacting of the three-dimensional filament-based porous structure with the second solvent with the purpose of causing phase inversion may thus be carried out while the filaments are being deposited.

According to a second preferred embodiment, contacting of the three-dimensional filament-based porous structure with the second solvent may be carried out after the filaments have been deposited and the three-dimensional filament-based porous structure has been formed. Contacting of the three-dimensional filament-based porous structure with the second solvent with the purpose of causing phase inversion may thus be carried out after deposition of the filaments has been terminated.

According to a third preferred embodiment, step d) may comprise a first step of contacting the three-dimensional filament-based porous structure formed in step c) with a third non-solvent for the one or more binders, preferably a non-solvent vapour, thereby subjecting the one or more binders to a partial phase inversion. This first step of step d) may be carried out in advance of contacting the three-dimensional filament-based porous structure with the second liquid solvent, after contacting the three-dimensional filament-based porous structure with the second liquid solvent, or simultaneously. The step of contacting the filament-based porous structure formed in step c) with the third non-solvent, in particular the non-solvent vapour, is preferably carried out in the course of filament deposition, in advance of step of contacting the filament-based porous structure with the second solvent. This way a partial phase inversion may be induced and some porosity may already be created in the filaments, to facilitate penetration of the second solvent and pore formation resulting therefrom. Also the partial phase inversion may impart some rigidity to the three-dimensional filament-based porous structure in an early stage. The step of contacting the porous structure with the third non-solvent may facilitate removal of the removable additive in a next step.

The present invention also relates to a three-dimensional macro-porous filament construct obtainable by the method described above, wherein the porosity formed by interconnected micropores comprises between 1 and 50%, preferably between 5 and 30% of the total porosity of the three-dimensional macro-porous filament construct, wherein said micropores consist of pores having a pore size equal to or smaller than 50µm, and wherein the filaments have an average surface roughness (Ra) higher than 4µm. The porosity of the macro-porous filament construct provided by macro-pores will generally comprises between 50 and 95 %, preferably between 60 and 85 % of the total porosity of the three-dimensional macro-porous filament construct, wherein said macropores have a pore size greater than 100µm.

The present invention further relates to the use of a three-dimensional macro-porous filament construct as described above, or a construct obtained by the above-described method for the manufacture of a catalyst, a sorbent, a chromatographic material.

The present invention further relates to a biomedical product such as a synthetic bone implant or bone graft, a tissue engineering scaffold, a drug-delivery device comprising a three-dimensional macro-porous filament construct as described above, or a three-dimensional macro-porous filament construct obtainable by the method described above. The biomedical product may comprise a bone promoting protein (BMP), stem cells, osteoblast cells, pharmaceuticals or/and a mixture thereof, which will usually be incorporated in the three-dimensional macro-porous filament construct after calcination and sintering has been carried out.

### Detailed Description.

The present invention provides a method based on 3D printing in combination with phase inversion for manufacturing a three-dimensional macro-porous construct having a suitable micro-porous filament morphology. The sintered construct obtained with a method according to the invention comprises filament struts with an interconnected micro-porous structure and roughened surfaces.

The term **"phase inversion"** as used herein involves the process of transforming a polymer, e.g. deposited using a mixture comprising the dissolved polymer in the form of filaments, in a controlled manner from a liquid to a solid state. This process comprises the making of a mixture of a predetermined material, for example in the form of a viscous paste, a suspension or a solution, to become the construct in a suitable solvent/phase inversion binder mixture, by deposition of filaments of the mixture in three dimensions in a desired shape, and exposing the thus printed 3D structure to a non-solvent for the phase inversion binder to cause the phase inversion binder to precipitate from the suspension or solution and form a three-dimensional filament construct in the desired shape. The phase inversion technique involves the precipitation or solidification of the phase inversion binder by exposing it to a non-solvent, so that a solid matrix is formed. The term "phase inversion" is well known in the art of producing porous constructs and for instance clearly defined in the textbook entitled "Basic Principles of membrane Technology", from Mulder M., Kluwer Academic Publishers, 1996.

The term **"porosity"** as used herein refers to a measure of the void spaces in a material after sintering and is measured herein as percent between 0 and 100%.

The terms **"macro-porosity"** or "macro-porous" or "highly porous" as used herein refer to porosity of the construct with macro-pores having a pore size greater than 50 µm in diameter. The terms "macro-porosity" or "macro-pores" are in some embodiments of the invention considered as synonyms. The term macro-porosity thus indicates that the construct of the invention has macro-pores having a pore size greater than 50 µm in diameter.

The term **"micro-porosity"** or "micro-porous" refers to porosity of the filaments as such after sintering and includes micro-pores having a pore size equal to or smaller than 50 µm. The terms "micro-porosity" or "micro-pores" are in some embodiments of the invention considered as synonyms. The term micro-porosity thus indicates that the filaments of the invention have micro-pores having a pore size equal to or smaller than 50 µm in diameter, and for instance having a pore size comprised between 0.1 and 50 µm, or between 0.5 and 50 µm, or between 1 and 30µm, and for instance lower than 40, 30, 20, 15, 10, 5, 1 µm.

The terms "strut filaments", "struts" or **"filaments""** are used herein as synonyms and refer to suspension or solution, as defined herein, that has been deposited, e.g. by (co)extrusion, in the form of filaments.

The term **"interconnected" micro-porosity** is used herein in reference to micro-porous filaments and indicates that the (micro)pores within various filaments that have been deposited according to a predetermined 3D pattern are in connection with each other, and as such provide paths, e.g. for gas or liquid transfer, within the construct, and between filaments according to the invention.

The term **"interconnected" macro-porosity** is used herein in reference to the macro-porous construct and indicates that the (macro)pores of the construct are in connection with each other, and as such provide paths, e.g. gas or liquid transfer, within the construct according to the invention.

The term **"roughening"** and its cognates are intended to refer to surface texture on the order of microns. Surface roughening is not intended to refer to larger features of an implant such as the thread.

The term **"surface roughness"** as used herein is also defined by the norm ISO 4287-1:1984 (Surface roughness -- Terminology -- Part 1: Surface and its parameters). Surface roughness can for instance be measured by non-contact, optical profilometry based on interferometry (VEECO, Wyko NT3300 - A.G. Olszak, J. Schmit, M.G. Heaton, "Interferometry: Technology and Applications," Veeco Instruments, Inc., 2650 E. Elvira Road, Tucson, AZ 85706, 2001).

With "Green structure" reference is made to a 3D printed structure of interconnected filaments that have not yet undergone a thermal treatment.

It shall be noted that in some embodiments of the present invention, the terms "construct" and "structure" and "scaffold" are used as synonyms. In some embodiments of the present invention, the terms "suspension", "solution" and "paste" are used as synonyms.

### A. Method

The present invention provides a method for producing a three-dimensional macro-porous filament construct comprising interconnected micro-porous filaments having a desired morphology.

### STEP a) and b)

A first step of the method of this invention comprises preparing a mixture, typically a suspension, in the form of a viscous paste or a suspension or a dispersion, comprising
- particles of one or more metals, one or more metal alloys or a mixture of one or more metals and one or more metal alloys,
- a first liquid solvent,
- optionally one or more binders soluble in the first liquid solvent.

Suitable metals or metal alloys may be selected from the following: aluminium, aluminium alloys; stainless steel alloys, including alloys of austenitic, ferritic and martensitic stainless steels; cobalt alloys; copper alloys; nickel alloys; silver alloys; gold alloys; platinum alloys, titanium, titanium alloys for example titanium-6aluminium-4-vanadium, zinc alloys, tantalum, tungsten, molybdenum, tantalum, tungsten, silver, molybdenum, iron, gold, platinum and stainless steel and combinations of two or more thereof. In a particularly preferred embodiment, the predetermined particulate material comprises particles of Ti or a Ti-alloy. In the description below, the method of this invention is described in reference to the use of particles of Ti and Ti-alloys.

Preferably, the metals or metal alloys have the form and shape of particles, which are typically characterized by a specific particle size distribution as well as a specific surface area. The particles may be provided in powder form. Within the scope of this invention the average particle diameter may be determined using laser diffraction. In an example the particles of the one or metals and one or more metal alloys may have an average particle diameter of maximum 2000 micron, the average minimal diameter will usually be 10 nm, but powder materials with a smaller or larger particle size may be used as well and the skilled person will be able to select the appropriate particle size depending on the intended use. Advantageously the particles have an average particle diameter which is equal to or less than 100 µm, more preferably less than 90 µm, most preferably less than 80 µm or less than 70 µm, in particular less than 60 µm or less than 50 µm. The particles preferably have an average particle size of at least 20 nm, preferably at least 25 nm or at least 30 nm, although the average particle size may be at least 1 µm as well.

According to preferred embodiments of the invention, the mixture formed in step a) may comprise at least 45 vol. %, preferably at least 50 vol. %, more preferably at least 55 vol. % or at least 60 vol. % of the particles of the one or more metals or metal alloys, with reference to the total volume of the mixture. It is further understood that the mixture of step a) preferably comprises at most 90 vol. %, preferably at most 85 vol. % and more preferably at most 80 vol. % of the particles of the one or more metals or metal alloys with reference to the total volume of the mixture. In a particularly preferred embodiment, the mixture of step a) comprises between 45 and 92 vol. %, preferably between 50 and 85 vol.% of the particles of the one or more metals or metal alloys with reference to the total volume of the mixture.

The **first liquid solvent** used in the method of this invention is a solvent for the one or more binders and serves to prepare the mixture so that it can take the form of a suspension, a viscous paste or a dispersion. Preferably the first solvent is a non-volatile liquid, a volatile liquid or a mixture of one or more volatile with one or more non-volatile solvents. The first liquid solvent may comprise one or more of an organic solvent, an inorganic solvent. Other suitable organic solvents include N-methyl-pyrrolidone (NMP), dimethyl-acetamide (DMAc), dimethylformamide (DMF), dimethylsulphoxide (DMSO), tetrahydrofurane (THF), s-caprolactam, 4-butyrolactone, methyl ethylketone, acetone, acetic acid, formylpiperidine, morfoline, chlorinated solvents for example chloroform, CCl4 and dioxane. Preferably said non-volatile liquid in said first solvent is N-methyl-2-pyrroliodone, and said volatile liquid in said first solvent is acetone.

In a preferred embodiment, the first liquid solvent is soluble in or miscible with the second solvent as defined herein.

The **second solvent** may be in a liquid or vapour phase. The second solvent is intended to be a non-solvent for the one or more binders contained in the mixture and is selected from those solvents capable of inducing phase inversion of the one or more binders upon contact with the second solvent. Solvents suitable for use as a second solvent may be selected from the list of solvents described above for the first solvent. It shall however be clear to the skilled person that the first solvent and the second solvent must be different from each other, and that the solubility of the one or more binders in the combination of the first and second solvent is lower than the solubility of the one or more binders in the first solvent. Additional solvents suitable for use as a second solvent include water, ionic liquids, aqueous solutions comprising an acid or base. The latter present the additional advantage that they may be capable of improving the solubility of the removable additive, depending on the nature of the additive. The acid may be a weak acid or a strong acid, for example acetic acid, HCl or any other acid considered suitable by the skilled person. The use of an acidic or alkaline solution may be preferred to permit fine tuning of the micro-porous structure of the filaments, especially in the course of the phase inversion step or in the step of removing of the removable additive to improve the solubility of the additive.

Advantageously the **one or more binders** may comprise one single phase inversion binder, or they may comprise a mixture of two or more phase-inversion binders. The one or more binders may comprise one or more rheology modification binders. The term **"phase inversion binder"** as used herein refers to a binder which is capable of undergoing phase inversion in a suitable non-solvent medium e.g. water, an alcohol, an acid, or a mixture thereof or in a suitable medium comprising a mixture of a non-solvent and a solvent for the phase inversion binder (see below). According to the present invention, the phase-inversion binder preferably comprises one or more polymers selected from the group comprising polysulphones, polyethersulphone, cellulose acetate, polyvinylidenefluorides, polyacrylonitriles, polyethylene-co-vinylalcohol, polyimides, polyether imides, polyamides and/or combinations thereof.

The term **"rheology modification binder"** as used herein refers to a binder which is capable of modifying the rheology or flow properties of the mixture of this invention. Rheology modification binders suitable for use in this invention may be binders which are pressure sensitive, i.e. the viscosity of which is reduced when subjected to pressure. According to the present invention, the rheology modification binder is preferably chosen from the group comprising hydrocolloids, cellulose derivates and/or combinations thereof.

In a preferred embodiment, the mixture prepared in step a) comprises a total amount of binder, i.e. phase-inversion binder and rheology modification binder taken together, of at least 0.5 vol.%, preferably at least 1 vol.%, more preferably at least 2 vol.%, even more preferably at least 3 vol.%, more preferably at least 4 vol.% and most preferably at least 5 vol.%, with respect to the total volume of the mixture. It is further understood that the mixture produced in step a) comprises a total amount of binder which is at most 30 vol.%, more preferably at most 28 vol.%, even more preferably at most 25 vol.%, more preferably at most 24 vol.%, even more preferably at most 22 vol.% and most preferably at most 20 vol.% with respect to the total volume of the mixture. The total amount of binder can for instance be 5, 7, 10, 12, 15, 18, 20, 22, 25 or 28 vol.% with respect to the total volume of the mixture, depending of the kind of the powder, e.g. its density, particle size, specific surface.

According to particular embodiments, the mixture obtained from step a) comprises between 0.5 vol.% and 30 vol.%, preferably between 1 and 25 vol.%, more preferably between 1 and 15 vol.% of a phase-inversion binder, and between 0 vol.% and 30 vol.%, and preferably between 0 and 15 vol.% of a rheology modification binder, with respect to the total volume of the mixture.

The invention provides a method wherein the **at least one removable additive** is dispersed in the mixture obtained from in step a) in the form of particles. Particles are understood to include solid particles, liquid droplets, for example droplets of a liquid solution or a gel. The at least one removable additive may therefore comprise particles of a solid material, or droplets of a liquid material or a gel, which are suitable for being dispersed in the mixture.

Dispersion of the at least one removable additive in the mixture containing the one or more metals and/or one or more metal alloys may be done simultaneously with preparing the mixture or it may be done in a separate step. In preferred embodiments, the volume ratio of the volume of said at least one removable additive to the volume of the particles of the predetermined material is at least 0.01:100, preferably at least 0.1:100, more preferably at least 1:100, even more preferably at least 5:100 and most preferably at least 10:100. It is further understood that said weight ratio of said at least one removable additive to said particles of a predetermined particulate material is at most 50:100, preferably at most 45:100, more preferably at most 40:100, even more preferably at most 35:100 and most preferably at most 30:100. By varying the amount or volume of additive that is incorporated into the mixture, the porosity of the three-dimensional macro-porous filament construct may be controlled as has been discussed above.

In a preferred embodiment, the skilled person may select the average particle size of the removable additive, thereby taking into account the envisaged pore volume and average pore diameter of the construct. It has namely been found that the pore diameter of the micropores may be varied by varying the size of the particles of the additive. Additionally, due to the presence of the at least one removable additive, a pore volume may be formed which exceeds the volume occupied by the removable additive. In an example, the particle size may range from about 0.01 micron up to 500 micron, preferably from about 0.05 micron up to 250 micron, more preferably up to 100 micron.

The particle size of the particles of one or more metals, one or more metal alloys as well as the particle size of the at least one removable additive is preferably selected such that it is sufficiently small for use with a particular extrusion nozzle, if extrusion is used as the filament deposition technique. The largest particles (d99 value) preferably have a diameter that is at least five times smaller, in particular at least ten times smaller, than the nozzle diameter.

According to an embodiment of the invention, the at least one removable additive is mainly insoluble in the first solvent (non-solvent for the phase inversion binder), but is soluble in removal agent.

According to an alternative embodiment the at least one removable additive is mainly insoluble in the first solvent (non-solvent for the phase inversion binder), but is soluble in the second solvent, wherein the second solvent is a non-solvent for the phase inversion binder. This way the additive may be at least partly removed after a partial phase inversion has been achieved. A preferred embodiment of the method of this invention comprises the step of contacting the at least one removable additive with the second solvent and dissolving at least part of the at least one removable additive in the second solvent.

The at least one removable additive is preferably selected such that it includes but is not limited to soluble polymers and inorganic salts capable of being dissolved in a solvent.

Inorganic salts suitable for use as an additive in the present invention include those which are soluble in an organic or inorganic solvent or in water, or in a mixture of one or more of these solvents comprising an acid or base to improve the solubility of the salt. Preferred salts include salts containing as a cationic part an alkali metal ion, an earth alkali metal ion, an earth metal ion, an ammonium salt or a mixture of two or more hereof. Particularly preferred salts contain sodium, potassium, calcium, magnesium, aluminium, or a mixture of two or more hereof as the cationic part. The anionic part of the at least one removable additive may include a nitrate, sulphate, phosphate, chloride, carbonate or halide, as well as mixtures of two or more hereof. Particularly preferred removable additives include one or more compounds selected from the group consisting of ionic liquids, NaCl, KCI and CaCO3 or a mixture of two or more hereof.

According to an alternative embodiment, the particles of the at least one removable additive comprise liquid droplets. Suitable examples of such removable additives include but are not limited to: solutions of polymers such as polyethylene glycol, polyvinylpyrolidone, polyvinyl alcohol, polylactic acid, polyethylene oxide etc. and mixtures of two or more hereof, in particular aqueous solutions of the afore-mentioned polymers.

According to a further alternative embodiment, the particles of said at least one removable additive comprises droplets of a suspension of the at least one removable additive in a dispersing agent.

The total volume % of organic compounds present in the mixture will generally depend on the density, the particle size distribution, the morphology and the specific surface of the particles of particles of the one or more metals and/or the one or more metal alloys. Preferably, the mixture is developed in order to minimize ash residue after thermal treatment. Preferably less than 6 vol.% of rheology modification binder, plasticizer and/or dispersant are incorporated into the mixture.

In an example, a mixture in the form of a powder paste can be prepared by mixing 20 to 85 vol.% of a Ti- or Ti-alloy powder with one or more binders and a sodium salt in a first liquid solvent in which the one or more binders are soluble.

In an embodiment of the invention, the mixture obtained in step a) has a viscosity of between 100 and 10000 Pa s at a shear rate of 1 s⁻¹.

The above described compositions can advantageously be used in the additive manufacturing or 3D printing of 3D objects, using filament deposition by extrusion of a viscous paste or suspension or dispersion (see description below method step b).

### STEP c)

A next step of the method of the present invention comprises forming the mixture obtained from step a) and b) into filaments and depositing the mixture in the form of filaments in a predetermined three-dimensional pattern thereby creating a three-dimensional filament-based porous green structure. The filaments will usually be deposited in stacked layers of filaments. The filaments within a layer may be positioned adjacent to each other or at a distance from each other. Filaments in a next layer will usually be positioned under an angle with respect to the filaments of the previous layer. That way filaments within different layers are interconnected and form a 3D object, with macro-pores between the filaments.

Deposition of the filaments may be carried out in several ways, depending on the nature of the composition and the intended application. Suitable 3D-printing technologies that allow to form filaments and to arrange these filaments in a three-dimensional porous structure include Fused Filament Fabrication (FFF), Direct Ink Writing (DIW; also known as (micro-)extrusion through a nozzle or an orifice), three-dimensional fibre or filament deposition (3D FD), and stereolithography, DLP (Digital Direct Light Processing (DLP), direct ink writing, micro-extrusion, robocasting etc. Of these, 3D-printing techniques involving filament deposition in multiple stacked layers are preferred, in particular three-dimensional fibre or filament deposition (3D FD) or micro-extrusion where the filament is obtained by extruding the mixture through a nozzle or by dispensing the mixture.

A first embodiment of filament deposition according to the invention comprises extrusion or dispensing of the mixture in the form of filaments and deposition of the filaments in three dimensions in a conventional environment, for example a non-reactive environment, which does not affect the composition and physical state of the filaments.

A second embodiment of filament deposition according to the invention comprises extrusion of the mixture and deposition of the filaments in a non-solvent environment for the one or more binders. The term "non-solvent environment" herewith refers to an environment with a relative non-solvent vapour for the one or more binders, more in particular for the at least one phase inversion binder, of at least 5 vol. %, and preferably of at least 10, 15, 20 or 25 vol. %. The non-solvent medium may be a humidifying environment or ambient air with a relative humidity of at least 50 %. In a preferred embodiment, said non-solvent environment is formed by a water vapour flow which contacts the three-dimensional filament-based porous structure, formed from step a) and b).

As mentioned above, deposition of the mixture obtained from step a) and b) in the form of filaments can be performed by means of different techniques, including extrusion of the prepared suspensions through nozzle(s). To that end, according to the invention, different types and configurations of nozzles can be used. Suitable types and configurations of nozzles are known in the state of the art, notably from EP2195131 B1, paragraphs [0073]-[0086] and [0089]-[0092] of which are incorporated herein by reference.

The present method includes the production of a 3D structure or scaffold or construct by controlled deposition of e.g. extruded filaments in a non-solvent environment for the one or more binders, following a planned or pre-set pattern.

### STEP d)

A next step in the method of this invention comprises subjecting the porous green structure formed in step c) to phase inversion. Phase inversion involves contacting the filaments formed in step c) with a second solvent with the purpose of inducing phase inversion of the one or more binders thereby creating a filament-based porous structure having a suitable filament morphology, whereby at least part of said filaments are transformed to a solid state

According to a first embodiment of step d) phase inversion of the one or more binders in the three-dimensional filament-based green porous structure formed in step c) may be induced by contacting the 3D printed structure obtained from in step c) with a second solvent, which is a non-solvent for the one or more binders, more in particular it is a non-solvent for the phase inversion binder.

According to a second embodiment of step d), phase inversion of the one or more binders in the three-dimensional filament-based porous structure may be achieved by contacting the green structure with a non-solvent for the one or more binders already during filament deposition in the course of step c). Causing phase inversion while the filaments are being deposited to form a 3D structure has the advantage that already in the course of filament deposition an immediate solidification of the filaments may be achieved, as well as formation of the pore structure. As a result, a structure with a higher rigidity and better shape retention ability may be obtained in an early stage of the process. Contacting with a non-solvent during filament deposition may for example be carried out by the use of a liquid non-solvent or by the use of a non-solvent vapour, using so-called Vapour Induced Phase inversion by flowing of a suitable non-solvent vapour over the filaments during deposition of the filaments (step d1).

If so desired, according to a third embodiment of step d), a combination of the first and second embodiment may be carried out in that the filaments are contacted with a non-solvent environment already during filament deposition, and the 3D printed green structure is contacted with a further non-solvent after printing of the 3D construct has been finalised. Thereby the non-solvent used during filament deposition may be the same material as the further solvent after the printing of the construct has been finalized, or it may differ therefrom. Differently said, the non-solvent vapour and liquid non-solvent may originate from the same solvent, for example water vapour and water or alcohol vapour and liquid alcohol. According to a different embodiment, the non-solvent vapour and liquid non-solvent may originate from different solvents. The second solvent may correspond either to the non-solvent or to the further non-solvent or to both.

According to an embodiment of the invention, the non-solvent vapour is blown over the filaments during filament deposition (step d1). After the treatment with the non-solvent vapour has been finalised, the thus obtained structure may be contacted with, for example be immersed in, a liquid non-solvent (step d2) with the purpose of inducing further phase-inversion, thereby creating a three-dimensional filament-based porous structure having suitable filament morphology.

Contacting the 3D printed structure with the non-solvent can be carried out in several ways, well known to the skilled person. Suitable methods include immersing the structure in, flowing of liquid over the filaments, as well as equivalent techniques known to the skilled person. Contacting of the 3D printed structure may involve Immersion Induced Phase inversion and thus creation of the micro-porous filament structure with specific filament surface morphology is performed by dipping or immersing or submerging a green 3D printed structure in a liquid non-solvent for the phase inversion binder (polymer) (step d2). Suitable liquid non-solvents include water, an alcohol, an acid, or a mixture of two or more thereof. Suitable examples of alcohols comprise but are not limited to methanol, ethanol, n-propanol, iso-propanol, n-butanol, octanol. Suitable examples of acids may comprise inorganic as well as organic acids, for example but not limited to acetic acid, citric acid, etc. A non-solvent/solvent mixture can also be used in this step. To optimize the morphology of the structure, addition of a (liquid) solvent as defined herein to the non-solvent can change the morphology and pore structure of the filaments and determine diffusion of the removable additive into the pores and therewith dissolution of the removable additive.

Advantageously, the at least one removable additive and the one or more binders may react differently to the second solvent. This may permit controlling solidification of the one or more binders and inducing additional pore formation by removal of the at least one removable additive, at least partly independently of each other. The phase inversion binder and the second solvent are preferably selected such that the phase inversion binder precipitates and solidifies upon contacting of the structure with the second solvent in step d). This solidified binder will usually be pyrolyzed in the thermal treatment of step f), thereby evacuating the micro-pores created by phase-inversion of the one or more binders.

### Step e)

The method of this invention further comprises a step e) for removing at least part of the removable additive from the phase inverted 3D structure.

According to a first embodiment of the invention the at least one removable additive is soluble in the second solvent. According to this first embodiment, phase inversion of the one or more binders and dissolution of at least part of the removable additive may both occur in step d). The invention provides for a method wherein micro-porosity is created by phase inversion of the one or more binders, and additional micro-porosity is created by removal of at least part of the at least one removable additive upon contacting the structure obtained from step c) with the second solvent. Contacting may for example by carried out by dipping or immersion, or any other method considered suitable by the skilled person.

According to an alternative embodiment, the solvent for dissolving the at least one removable additive may comprise the second solvent which is rendered more acidic or more alkaline by addition of an acid or base to the second solvent having the green structure immersed therein. In such embodiments, micro-porosity is created by phase inversion of the one or more binders, and additional micro-porosity is created upon acidification of the second solvent or increasing the pH of the second solvent to remove at least part of the at least one removable additive from the green structure, for example by dipping or immersion.

According to a further alternative embodiment, said at least one removable additive is not soluble in the second solvent, is poorly soluble therein or has been partly dissolved upon contact with the second solvent in step d). The method according to such an embodiment will then usually further comprise an additional step e) of contacting the phase inverted structure obtained from step d) with a third solvent for the at least one removable additive with the purpose of dissolving the at least one removable additive. Thereby contacting may for example involve dipping or immersing or any other method considered suitable by the skilled person. Again, as the removable additive is dissolved from the phase inverted structure, additional micro-porosity is created, prior to the calcination step.

According to a still further embodiment, the third solvent for dissolving the at least one removable additive may be an acidic medium or an alkaline medium and is capable of dissolving the at least one removable additive.

The method of this invention presents the additional advantage that an unexpected level of additional micro-porosity can be formed on top of the micro-porosity induced by phase inversion of the one or more binders, by removal of the at least one removable additive without increasing brittleness of the three-dimensional macro-porous filament construct. This is important since the amount of the one or more binders that can be incorporated into the mixture is limited, because the decomposition of the phase inverted one or more binders gives rise to the formation of carbonaceous materials that are left behind in the construct. This leads to embrittlement of the construct and brittleness may increase to undesired levels.

It will be clear that at least the following parameters affect the phase inversion process and allow hence tailoring the filament structure:
- composition of the mixture (including but not limited to the nature and portion of the components, particle size and particle size distribution of the predetermined particulate material and the at least one removable additive);
- extrusion parameters, including initialization of phase inversion by use of a non-solvent vapour;
- rate of dipping and residence time of the green structure in the second solvent, type of non-solvent, addition of a solvent to said non-solvent and temperature of the solvent (or solvent mixture);
- solubility characteristics of said at least one removable additive by said second solvent;
- type of a third liquid solvent and solubility characteristics of said at least one removable additive by said third liquid solvent.

### STEP f)

In a further step of the method of the present invention, the phase inverted structure obtained from step e) is subjected to a thermal treatment. The thermal treatment may involve drying of the phase inverted structure obtained from step e), but it may also involve an additional calcination and/or sintering of the phase inverted structure obtained from step e).

Preferably, the structure obtained from step e) is dried prior to being subjected to calcination and/or sintering. Drying may for instance be done at room temperature using air drying or in a controlled atmosphere (temperature, humidity). Drying, calcination and sintering may be carried out in the same or in a different environment. Similarly to drying, calcination and/or sintering may be carried out in air or in a controlled atmosphere, and temperature and humidity of the atmosphere may be selected taking into account the nature of the material and the envisaged porosity and degree of crimp or shrinkage.

The method of this invention may further comprise a calcination step which comprises a heating step wherein the green structure obtained after step e) is heated at a rate between 5 to 50°C/hour to a temperature between 200 and 600°C, and for instance between 400 and 600°C, and preferably below 500°C. Preferably, especially in the case of the use of Ti or Ti-alloy powder particles in the suspension, calcination is performed under an inert atmosphere such as argon or at low pressure, preferably at a pressure of less than 10⁻³mbar. During this step most of the organic material will be pyrolyzed.

The present method may further comprise a pre-sintering step between the calcination step and the sintering step, comprising heating of the construct to a temperature of between 900 and 1000°C. In the case where the mixture contains Ti or Ti-alloy, pre-sintering results in a structure where the Ti or Ti-alloy powder particles start to sinter together, allowing better handling of the structure. Preferably, this pre-sintering step is performed in an inert atmosphere or under a vacuum of at least 10⁻⁴ mbar on a Y₂O₃-coated substrate.

Next, and especially in the case the mixture contains Ti or Ti-alloy particles, the obtained structure is sintered on a Y₂O₃-coated substrate or on a Y₂O₃ powder bed, allowing a further shrinking of the structure. An inert atmosphere or a high vacuum of more than 10⁻⁴ mbar may be used. Sintering is preferably carried out by heating the three-dimensional phase inverted structure at a heating rate of between 1 to 10°C /min to a temperature of between 1000 and 1500°C, and for instance of between 1200 and 1500°C and keeping said structure at said temperature for a pre-determined period of time of between 1 to 5 hours, where after a cooling to room temperature follows at a rate of e.g. 20°C/min.

Depending on the dimension requirements, one or more machining steps may be carried out, e.g. machining of the thus obtained 3D construct, machining after pre-sintering etc. can be performed. Machining may for example include one or more of drilling, cutting, tumbling etc.

While the invention has been described hereinabove with reference to specific embodiments, this is done to illustrate and not to limit the invention, the scope of which is defined by the accompanying claims. The skilled person will readily appreciate that different combinations of features than those described herein are possible without departing from the scope of the claimed invention.

The construct of this invention may be used for the manufacture of a (bio)medical product, such as a synthetic bone implant or bone graft, a tissue engineering scaffold, a drug-delivery device. Thereto, the construct may contain comprising a bone promoting protein (BMP), stem cells, osteoblast cells, pharmaceuticals or/and a mixture thereof.

The invention further relates to a biomedical product such as a synthetic bone implant or bone graft, a tissue engineering scaffold, a drug-delivery device comprising a construct as described above.

The invention further relates to the use of a construct that may be obtained from the method of this invention for the manufacture of a catalyst, a sorbent, a chromatographic material, a heat exchanger.

### Brief Description of the Figures

These and other features and advantages of embodiments of the present invention will now be described in more detail with reference to the accompanying drawings, in which:
- Figure 1 schematically illustrates the different steps occurring in the method of this invention.
- Figure 2 shows the evolution of the micro-porosity as a function of the amount of phase inversion binder in the mixture that is to be deposited as filaments, for a prior art composition.

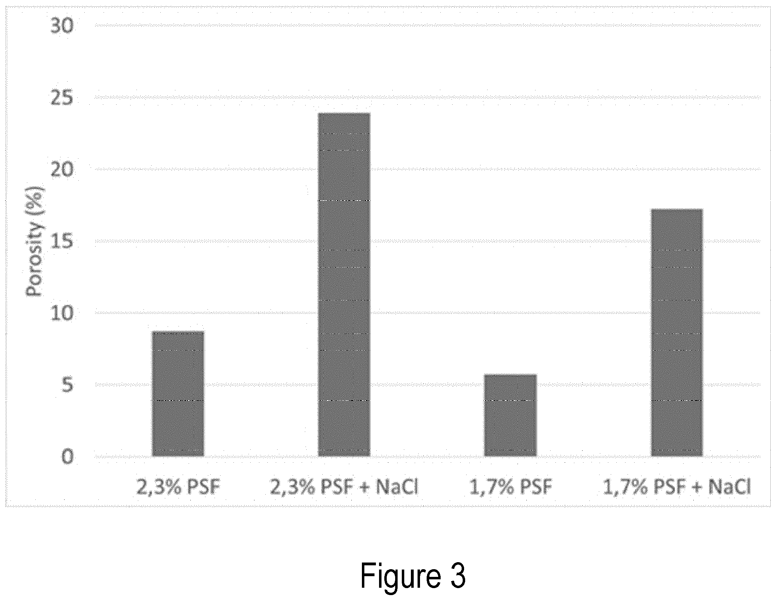

- Figure 3 shows the porosity of a 3D construct, using varying amounts of phase inversion binder and varying amounts of NaCl as removable additive.
- Fig. 4a - 4b illustrate electron microscopy images of 3D constructs obtained from a mixture containing Ti and 2.3 wt. % of polysulfone phase inversion binder, respectively with and without addition of NaCl as removable additive.
- Fig. 5a - 5b illustrate electron microscopy images of 3D constructs obtained from a mixture containing Ti and 1.7 wt. % of polysulfone phase inversion binder, respectively with and without addition of NaCl as removable additive.

### Experimental Section

Porosity within the filaments (micro-porosity) is measured using image analysis on SEM pictures. Based on grey value differences, the software (KS 400, C. Zeiss) is able to count the ratio of pixels representing porosity and pixels representing material.

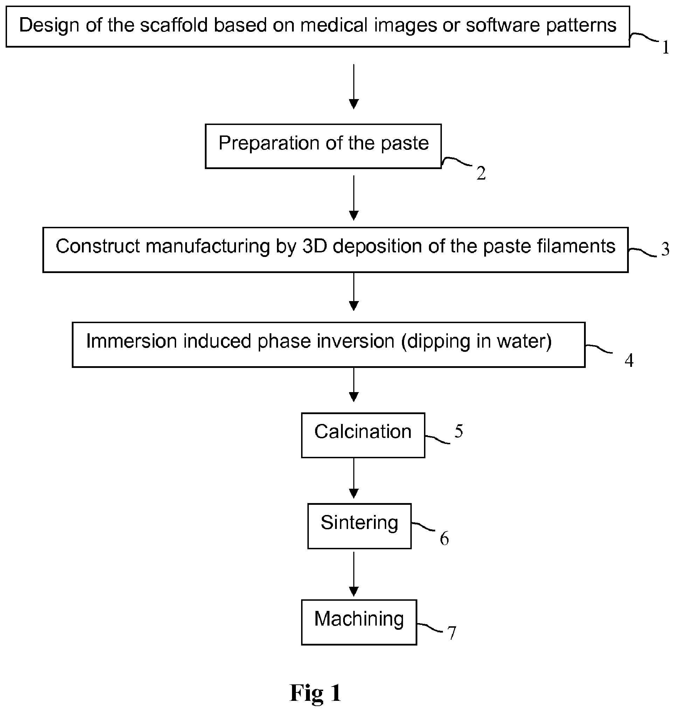

A 3D structure or scaffold was built by extrusion of filaments of a viscous paste using the following steps, as illustrated in FIG. 1:
- Based on images or predefined software patterns, a near net shape design of a scaffold is made in box 1, taking into account the shrinkage of the scaffold during the sintering. If so desired, some over-dimensioning or simplification of the shape can be foreseen, allowing the use of final machining in order to obtain the required dimensions and tolerances.
- a viscous paste is prepared in box 2 by e.g. mixing a powder of particles of a predetermined material in the indicated amount with a mixture of phase inversion binder, removable additive, and solvent. As a binder use is made of a mixture of a phase-inversion binder and a rheology modification binder.
- The paste is brought in a dispending unit comprising a syringe vessel and a nozzle. The unit is mounted on a CNC machine and connected with an air pressure plunger so that the flow rate of the paste can be controlled.
- The CNC-machine is programmed to cause the nozzle to move and deposit filaments according to a well-defined pattern and within a well-defined form. The CNC machine is programmed to continuously deposit the filaments layer by layer in a predefined pattern. Depending on the desired thickness of the filaments a nozzle with a corresponding diaphragm opening will be chosen e.g. between 0.1 and 2 mm. The deposition parameters, e.g. the distance between the nozzle and the surface of the structure, non-solvent environment for vapour induced phase inversion to solidify the filaments, the speed of the nozzle movement, the air pressure and the temperature and airflow of the environment, etc.. are controlled. A 3D-structure is built in box 3 by depositing the filaments layer by layer according to the programmed pattern and according to the required dimensions.
- Micro-porosity of the filament struts is created by contacting the filaments with the second non-solvent environment (box 4) whereafter the scaffold is dried
- then the structure undergoes thermal treatment by calcination under inert atmosphere or vacuum in box 5 and sintering under inert atmosphere or vacuum in box 6
- Depending of dimension requirements a final machining step can be performed in box 7.

### Comparative experiments I-IV.

A 3D green structure was made using filament micro extrusion of a suspension comprising Ti powder (55 - 95 wt. %) and a density of 4.43 g/cm³, ethyl cellulose (0.1- 3 wt. %), N-methyl pyrrolidone (5 - 30 wt. %) with a density of 1.03 g/cm³. The amount of polysulfone phase inversion binder having a density of 1.25 g/cm³ was varied between 0.5 - 20 wt. % (on Ti basis).

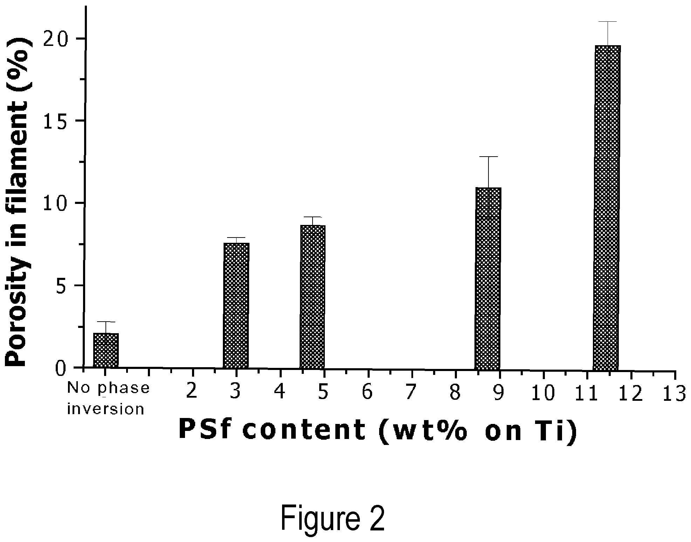

Porosity within the filament (micro-porosity) was measured using image analysis on SEM pictures. Based on grey value differences, the software (KS 400, C. Zeiss) is able to count the ratio of pixels representing porosity and pixels representing material. FIG. 2a illustrates the influence of the amount of a polysulphone phase inversion binder (in vol% PSf) on micro-porosity (in %) of filaments in a construct according to the invention. Increasing the amount of phase inversion binder in the suspension that is to be deposited causes an increase in micro-porosity within the filaments (see FIG. 2).

### Example 1-5. Varying amount of phase inversion binder and additive.

A 3D construct was made by 3D filament deposition of a viscous powder paste having the following composition :
- 125 g of Ti powder, with an average particle diameter of maximum 45 micron and a density of 4.43 g/cm³
- 16.6 g N-methyl pyrrolidone with a density of 1.03 g/cm³
- Respectively 2.9 g - 2.24 g and 0 g of polysulfone phase inversion binder having a density of 1.25 g/cm³
- Respectively 0 g, 2.0 g and 4.0g of NaCl as water soluble additive, with a d90 of 6 micron and a density of 2.16 g/cm³
The volume ratio of the phase inversion binder to the amount of Ti, and the volume ratio of NaCl as removable additive to the amount of Ti were varied as indicated in table 1 below. The 3D printed construct was dried and subjected to calcination at 1350°C as described above.

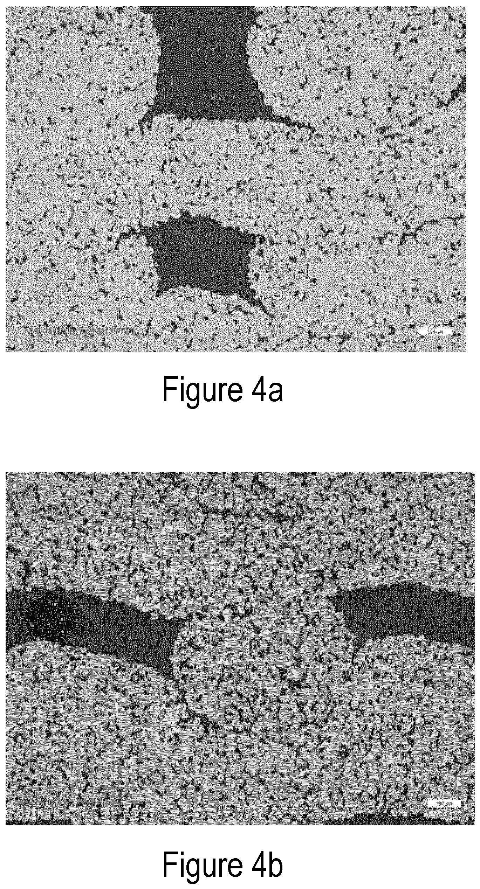

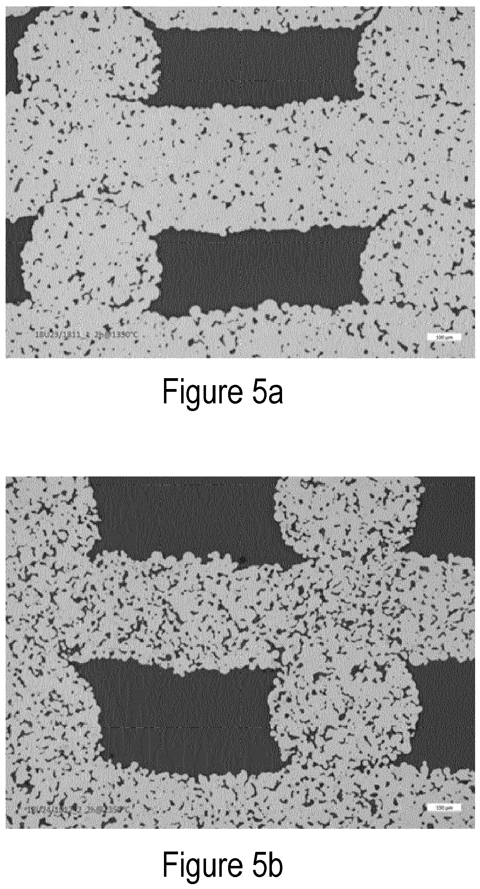

Micro-porosity of examples 1-4 was measured using image analyses as described above. The results are illustrated in fig. 4 and 5. Fig. 4a - 4b illustrate electron microscopy images of 3D constructs obtained using the above-mentioned compositions, in particular of example 1, 2. Fig. 5a and 5b illustrate electron microscopy images of 3D constructs obtained in example 4 and 5.

**Table 1.**

| | Wt.% phase inversion binder in mixture | Volume ratio % NaCL/ % Ti powder | Micro-porosity vol. % image analysis | Theoretical micro-porosity vol. % |
|---|---|---|---|---|
| 1 | 2.3 | 0 | 8.7 | |
| 2 | 2.3 | 3.16 | 23.9 | 15.2 |
| 3 | 1.7 | 0 | 5.7 | |
| 4 | 1.7 | 3.16 | 17.2 | 11.5 |
| 5 | 1.7 | 6.12 | 17.6 | 11.9 |

From the comparison of fig. 4a and 4b, respectively fig.5a and 5b, it can be observed that addition of NaCl as removable additive, gives rise to the formation of additional micro-pores in the filaments. Mercury porosimetry measurements illustrate that the pore system is accessible, and that the pores are interconnected.

## Claims

1. A method for producing a three-dimensional macro-porous filament construct having interconnected microporous filaments and a suitable morphology, said method comprising the steps of:
a) preparing a mixture comprising particles of one or more metals, one or more metal alloys or a mixture of the afore mentioned materials, one or more binders, a first liquid solvent for the one or more binders, and optionally one or more dispersants;
b) dispersing particles containing at least one removable additive in said mixture;
c) depositing said mixture in the form of filaments in a predetermined three-dimensional pattern of interconnected filaments, thereby creating a three-dimensional filament-based porous green structure,
d) contacting the three-dimensional filament-based porous green structure formed in step c) with a second solvent which is a non-solvent for the one or more binders, with the purpose of inducing phase inversion thereby creating a filament-based phase inverted porous structure having a suitable filament morphology, whereby at least part of said filaments are transformed to a solid state,
e) contacting the phase inverted structure with a removal agent for removing at least part of the at least one removable additive,
f) subjecting the thus obtained structure to a thermal treatment.

2. The method according to claim 1, wherein the thermal treatment involves calcination and sintering of the structure.

3. The method according to claim 1 or 2, wherein step d) comprises the steps of:
d1) d1) inducing phase inversion by bringing said filaments during the deposition of the filaments into contact with a non-solvent vapour, and
d2) d2) completing phase inversion by immersing the structure of step d1) in the second liquid non-solvent, thereby creating a filament-based porous structure having suitable filament morphology.

4. The method according to any of the previous claims, wherein said at least one removable additive comprises particles of one or more of the group of polymers, inorganic salts which are solvent soluble.

5. The method according to any one of claims 1-3, wherein the particles of said at least one removable additive comprises droplets of a suspension of the at least one removable additive in a dispersing agent.

6. The method according to any one of claims 1-3, wherein said at least one removable additive comprises droplets comprising one or more liquids, preferably selected from the group comprising polyethylene glycol, polyvinylpyrolidone, polyvinyl alcohol, polylactic acid, polyethylene oxide etc. and mixtures of two or more hereof.

7. The method according to any one of the previous claims, wherein the size of the particles of the at least one removable additive ranges from about 0.01 micron up to 500 micron, preferably from about 0.05 micron up to 250 micron, more preferably from about 0.05 micron up to 100 micron.

8. The method according to any one of claims 1-3, wherein step e) comprises contacting the structure obtained from step d2) with a third liquid solvent which is a solvent for the at least one removable additive so as to at least partly dissolve the removable additive.

9. The method according claim 8, wherein the method further comprises adding an acid or a base to the third liquid solvent to improve solubility of the said at least one removable additive.

10. The method according to any one of the previous claims, wherein the second liquid solvent is a solvent for the at least one removable additive.

11. The method according to any one of the previous claims, wherein the volume ratio of said at least one removable additive to the particles of the one or more metals and/or metal alloys is at least 0.01:100, preferably at least 0.1:100, more preferably at least 1:100, most preferably at least 5:100, in particular at least 10:100.

12. The method according to any one of the previous claims, wherein the volume ratio of said at least one removable additive to the one or more metals and/or metal alloys is at most 50:100, preferably at most 45:100, more preferably at most 40:100, even more preferably at most 35:100 and most preferably at most 30:100.

13. The method according to any one of the previous claims, wherein the mixture of step a) comprises at least 45 vol. %, preferably at least 50 vol. %, more preferably at least 55 vol. % or at least 60 vol. % of the particles of the one or more metals or metal alloys with reference to the total volume of the mixture.

14. The method according to any one of the previous claims, wherein the mixture of step a) comprises at most 90 vol. %, preferably at most 85 vol. %, more preferably at most 80 vol. % the particles of the one or more metals or metal alloys with reference to the total volume of the mixture, most preferably between 50 and 85 vol.%.

15. A three-dimensional macro-porous filament construct obtainable by the method according to any of claims 1-14, wherein the porosity formed by interconnected micropores comprises between 1 and 50%, preferably between 5 and 30% of the total porosity of the three-dimensional macro-porous filament construct, wherein said micropores consist of pores having a pore size equal to or smaller than 50µm, and wherein the filaments have an average surface roughness (Ra) higher than 4µm.

16. The three-dimensional macro-porous filament construct according to claim 15 wherein the porosity of said macro-porous filament construct provided by macro-pores comprises between 50 and 95 %, preferably between 60 and 85 % of the total porosity of the three-dimensional macro-porous filament construct, wherein said macropores have a pore size greater than 100µm.

17. A biomedical product such as a synthetic bone implant or bone graft, a tissue engineering scaffold, a drug-delivery device comprising a three-dimensional macro-porous filament construct as claimed in claim 15 or 16, or a three-dimensional macro-porous filament construct obtainable by the method of any one of claims 1-14.
